(19) Europäisches Patentamt
European Patent Office
Office européen des brevets

(11) Veröffentlichungsnummer: **0 085 843**
**B1**

(12) # EUROPÄISCHE PATENTSCHRIFT

(45) Veröffentlichungstag der Patentschrift: **04.09.85**

(51) Int. Cl.[4]: **A 61 K 31/41,** A 61 K 31/415 // C07D249/08, C07D233/60

(21) Anmeldenummer: **83100260.5**

(22) Anmeldetag: **14.01.83**

(54) **Antimykotisches Mittel.**

(30) Priorität: **27.01.82 DE 3202602**

(43) Veröffentlichungstag der Anmeldung: **17.08.83 Patentblatt 83/33**

(45) Bekanntmachung des Hinweises auf die Patenterteilung: **04.09.85 Patentblatt 85/36**

(84) Benannte Vertragsstaaten: **BE CH DE FR GB IT LI LU NL SE**

(56) Entgegenhaltungen:
**EP - A - 0 043 419**
**EP - A - 0 052 424**
**EP - A - 0 054 974**
**US - A - 4 123 542**
**US - A - 4 277 475**

(73) Patentinhaber: **BAYER AG, Konzernverwaltung RP Patentabteilung, D-5090 Leverkusen 1 Bayerwerk (DE)**

(72) Erfinder: **Holmwood, Graham, Dr., Katernbergerstrasse 184, D-5600 Wuppertal 1 (DE)**
Erfinder: **Regel, Erik, Untere Bergerheide 26, D-5600 Wuppertal 1 (DE)**
Erfinder: **Jäger, Gerhard, Dr., Gellertstrasse 18, D-5090 Leverkusen 1 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**

EP 0 085 843 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft antimykotische Mittel mit einem Gehalt an neuen Ether-Derivaten von substituierten 1-Hydroxyalkyl-azolen.

Es ist bereits bekannt geworden, dass bestimmte 1-Hydroxy-ethylazol-Derivate, wie beispielsweise 2-(4-Chlor-2-methyl-phenoxymethyl)- und 2-(4-Methylphenoxymethyl)-3,3-dimethyl-1-(1,2,4-triazol-1-yl)-butan-2-ol oder 2-(4-Chlorphenoxymethyl)-3,3-dimethyl-1-(imidazol-1-yl)-butan-2-ol bzw. 4,4-Dimethyl-3-(imidazol-1-yl)-1-(2-methylphenyl)-pentan-3-ol, gute antimykotische Eigenschaften aufweisen (vergleiche DE-A Nr. 3018865).

Es wurde gefunden, dass die neuen Ether-Derivate von substituierten 1-Hydroxyalkyl-azolen der allgemeinen Formel:

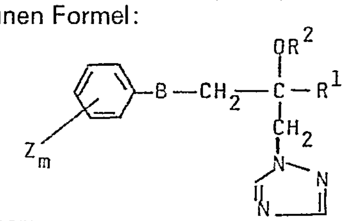 (I)

in welcher:

B für Sauerstoff, Schwefel oder die $CH_2$-Gruppe steht,

$R^1$ für die Gruppierungen

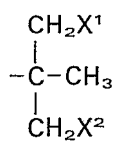 und 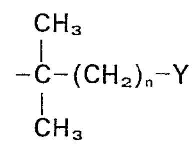

steht, oder für gegebenenfalls durch Alkyl mit 1 bis 2 Kohlenstoffatomen substituiertes Cycloalkyl mit 3 bis 7 Kohlenstoffatomen und für gegebenenfalls substituiertes Phenyl steht, wobei als Substituenten genannt seien:

Halogen, Alkyl mit 1 bis 4 Kohlenstoffatomen sowie Halogenalkyl mit 1 bis 2 Kohlenstoff- und 1 bis 5 gleichen oder verschiedenen Halogenatomen,

$X^1$ für Wasserstoff oder Halogen steht,

$X^2$ für Halogen steht,

Y für Alkyl, Alkoxy, Alkylthio, Halogenalkoxy, Halogenalkylthio, Alkenyl mit bis zu 6 Kohlenstoffatomen, Alkoxycarbonyl mit 1 bis 4 Kohlenstoffatomen im Alkylteil, Cyano sowie für gegebenenfalls substituiertes Phenyl, Phenoxy, Phenylthio, Phenylalkoxy mit 1 bis 4 Kohlenstoffatomen im Alkylteil steht, wobei jeweils als Phenylsubstituenten genannt seien:

Halogen, Alkyl mit 1 bis 4 Kohlenstoffatomen; Alkoxy und Alkylthio mit jeweils 1 bis 2 Kohlenstoffatomen; Halogenalkyl, Halogenalkoxy und Halogenalkylthio mit jeweils 1 bis 5 gleichen oder verschiedenen Halogenatomen; Cyclohexyl, Dialkylamino mit 1 bis 4 Kohlenstoffatomen in jedem Alkylteil, Nitro, Cyano sowie Alkoxycarbonyl mit 1 bis 4 Kohlenstoffatomen im Alkylteil,

n für die Zahlen 0, 1 oder 2 steht,

Z für Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen sowie für jeweils gegebenenfalls durch Halogen und Alkyl mit 1 bis 2 Kohlenstoffatomen substituiertes Phenyl, Phenoxy und Phenylalkyl sowie Phenylalkoxy mit 1 bis 2 Kohlenstoffatomen im Alkylteil bzw. im Alkoxyteil steht,

$R^2$ für geradkettiges oder verzweigtes Alkyl mit 1 bis 4 Kohlenstoffatomen, für geradkettiges oder verzweigtes Alkenyl und Alkinyl mit jeweils 2 bis 4 Kohlenstoffatomen sowie für gegebenenfalls substituiertes Phenyl oder Benzyl, wobei als Phenylsubstituenten die bei Y bereits genannten in Frage kommen, und

m für die Zahlen 0, 1, 2 oder 3 steht,

sowie deren Säureadditionssalze gute antimikrobielle, insbesondere antimykotische Eigenschaften aufweisen.

Die Verbindungen der Formel (I) besitzen ein asymmetrisches Kohlenstoffatom und können deshalb in den beiden optischen Isomerenformen anfallen.

Überraschenderweise zeigen die erfindungsgemässen Ether-Derivate von substituierten 1-Hydroxyalkyl-azolen der Formel (I) ein besseres antimykotisches Wirkungsspektrum als die aus dem Stand der Technik bekannten Verbindungen 2-(4-Chlor-2-methylphenoxymethyl)- und 2-(4-Methylphenoxymethyl)-3,3-dimethyl-(1,2,4-triazol-1-yl)-butan-2-ol oder 2-(4-Chlorpehnoxymethyl)-3,3-dimethyl-1-(imidazol-1-yl)-butan-2-ol bzw. 4,4-Dimethyl-3-(imidazol-1-yl)-1-(2-methylphenyl)-pentan-3-ol, welche chemisch und wirkungsmässig naheliegende Verbindungen sind.

Bevorzugt sind diejenigen Verbindungen der Formel (I), in denen:

$R^1$ für geradkettiges oder verzweigtes Alkyl mit 1 bis 4 Kohlenstoffatomen steht, und

B, $R^2$, Z und m für die oben genannten bevorzugten Bedeutungen stehen.

Besonders bevorzugt sind diejenigen Verbindungen der Formel (I), in denen

$R^1$ für die Gruppierungen

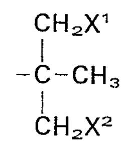 und 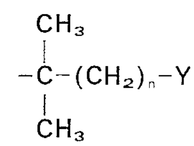

steht, oder für jeweils gegebenenfalls durch Methyl oder Ethyl substituiertes Cyclopropyl, Cyclopentyl und Cyclohexyl steht sowie für gegebenenfalls durch Fluor, Chlor, Methyl oder Trifluormethyl substituiertes Phenyl steht,

$X^1$ für Wasserstoff, Fluor, Chlor oder Brom steht,

$X^2$ für Fluor, Chlor oder Brom steht,

Y für Methyl, Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für gegebenenfalls substituiertes Phenyl, Phenoxy, Phenylthio, Phenylmethoxy und Phenylmethylthio steht, wobei jeweils als Phenylsubstituenten genannt seien: Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl,

n für die Zahlen 0, 1 oder 2 steht,

Z für Fluor, Chlor, Brom, Methyl, tert.-Butyl, Cyclohexyl, Methoxy, Methylthio, Trifluormethyl, Trifluormethoxy, Trifluormethylthio sowie für gegebenenfalls durch Fluor, Chlor und Methyl substituiertes Phenyl, Phenoxy, Benzyl oder Benzyloxy steht,

$R^2$ für Methyl, Ethyl, n-Propyl, Isopropyl, n-Butyl, Isobutyl, Allyl, Propargyl sowie für gegebenenfalls substituiertes Phenyl oder Benzyl steht, wobei als Phenylsubstituenten genannt seien: Fluor, Chlor, Methyl, Ethyl, Methoxy, Methylthio, Trifluormethyl, Trifluormethoxy und Trifluormethylthio,

B und m für die in der Erfindungsdefinition angegebene Bedeutung stehen.

Besonders bevorzugt sind ausserdem diejenigen Verbindungen der Formel (I), in denen:

$R^1$ für tert.-Butyl, Isopropyl oder Methyl steht, und

B, $R^2$, Z und m für die oben genannten besonders bevorzugten Bedeutungen stehen.

Bevorzugte erfindungsgemässe Verbindungen sind auch Additionsprodukte aus Säuren und denjenigen Ether-Derivaten von substituierten 1-Hydroxyalkyl-azolen der Formel (I), in denen die Substituenten A, B, $R^1$, $R^2$ und $Z_m$ die Bedeutungen haben, die bereits vorzugsweise für diese Substituenten genannt wurden.

Zu den Säuren, die addiert werden können, gehören vorzugsweise Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure und Milchsäure sowie Sulfonsäuren, wie z. B. p-Toluolsulfonsäure und 1,5-Naphthalindisulfonsäure.

Die erfindungsgemäss zu verwendenden Wirkstoffe und deren Säureadditionssalze sind noch nicht bekannt. Sie können jedoch in einfacher und bekannter Art und Weise erhalten werden, indem man Alkoholate von 1-Hydroxy-alkyl-azolen der Formel:

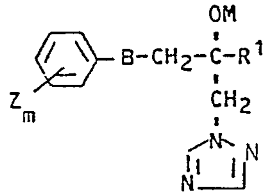

(II)

in welcher:

B, $R^1$, Z und m die oben angegebene Bedeutung haben, und

M für ein Alkalimetall, eine quarternäre Ammonium- oder Phosphoniumgruppe steht, mit einem Halogenid der Formel:

$$R^2-Hal \qquad (III)$$

in welcher:

$R^2$ die oben angegebene Bedeutung hat und

Hal für Halogen steht,

in Gegenwart eines inerten organischen Lösungsmittels, wie beispielsweise Dioxan, bei Temperaturen zwischen 20 und 100°C umsetzt. Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt; gegebenenfalls wird Säureadditions-Salz hergestellt.

In einer bevorzugten Ausführungsform wird zweckmässigerweise so verfahren, dass man von einem substituierten 1-Hydroxy-alkyl-azol-Derivat der Formel (IV) ausgeht, letzteres in einem geeigneten inerten organischen Lösungsmittel mittels Alkali-metall-hydrid oder Alkalimetall-amid in das Alkalimetall-alkoholat der Formel (II) überführt, und letzteres ohne Isolierung sofort mit einem Halogenid der Formel (III) umsetzt, wobei unter Austritt von Alkalihalogenid die erfindungsgemässen Verbindungen der Formel (I) in einem Arbeitsgang erhalten werden.

Nach einer weiteren bevorzugten Ausführungsform werden zweckmässigerweise die Herstellung der Alkoholate der Formel (II) sowie die erfindungsgemässe Umsetzung in einem Zweiphasensystem, wie beispielsweise wässerige Natron- oder Kalilauge/Toluol oder Methylenchlorid, unter Zusatz von 0,01-1 mol eines Phasen-Transfer-Katalysators, wie beispielsweise Ammonium- oder Phosphoniumverbindungen, durchgeführt, wobei in der organischen Phase oder an der Grenzfläche die Alkoholate entstehen und mit den in der organischen Phase befindlichen Halogeniden umgesetzt werden.

Die Säureadditionssalze der Verbindungen der Formel (I) können in einfacher Weise nach üblichen Salzbildungsmethoden, z. B. durch Lösung einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die Alkoholate der Formel (II) sind noch nicht bekannt. Sie können jedoch in allgemein bekannter Art und Weise erhalten werden, indem man die entsprechenden substituierten 1-Hydroxyalkylazole der Formel:

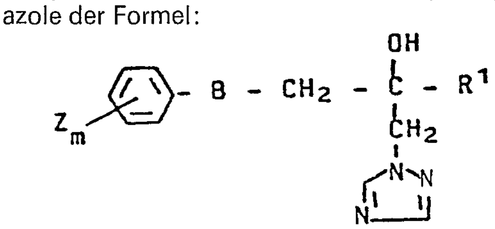

(IV)

in welcher:

B, $R^1$, Z und m die oben angegebene Bedeutung haben,

mit geeigneten starken Basen, wie Alkalimetallamiden oder -hydriden, quarternären Ammoniumhydroxiden oder Phosphoniumhydroxiden in einem indifferenten Lösungsmittel, wie beispielsweise Dioxan, bei Raumtemperatur umsetzt.

Die substituierten 1-Hydroxyalkyl-azole der Formel (IV) sind teilweise bekannt (vergleiche DE-OS Nr. 3018866); teilweise sind sie Gegenstand einer parallelen Patentanmeldung. Sie werden erhalten, indem man Oxirane der Formel:

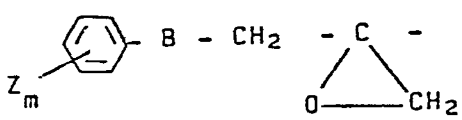

(V)

in welcher:

B, $R^1$, Z und m die oben angegebene Bedeutung haben,

mit Triazol der Formel:

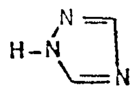

(VI)

in Gegenwart eines inerten organischen Lösungsmittels, wie beispielsweise Ethanol, und gegebenenfalls in Gegenwart einer Base, wie beispielsweise Natriumethylat, gegebenenfalls unter einem Druck von 1 bis 24 bar, bei Temperaturen zwischen 60 und 150°C umsetzt, oder Azolylmethyloxirane der Formel:

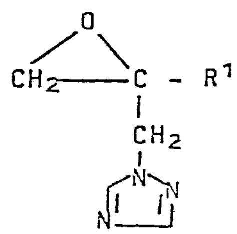

(VII)

in welcher:

$R^1$ die oben angegebene Bedeutung hat,

mit (Thio)Phenolen der Formel:

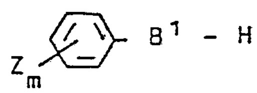

(VIII)

in welcher:

Z und m die oben angegebene Bedeutung haben und

$B^1$ für Sauerstoff oder Schwefel steht,

in Gegenwart eines inerten organischen Lösungsmittels, wie beispielsweise Ethanol, und gegebenenfalls in Gegenwart einer Base, wie beispielsweise Natriumethylat, gegebenenfalls unter einem Druck von 1 bis 25 bar, bei Temperaturen zwischen 60 und 100°C umsetzt.

Die Oxirane der Formel (V) sind teilweise bekannt (vergleiche DE-OS Nr. 3018866), teilweise sind sie Gegenstand einer parallelen Patentanmeldung. Sie werden erhalten, indem man Ketone der Formel:

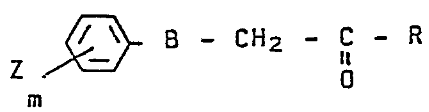

(IX)

in welcher:

B, R, Z und m die oben angegebene Bedeutung haben, entweder

α) mit Dimethyloxosulfonium-methylid der Formel:

$$\overset{\delta^+}{(CH_3)_2SO}\overset{\delta^-}{CH_2} \qquad (X)$$

in an sich bekannter Weise in Gegenwart eines Verdünnungsmittels, wie beispielsweise Dimethylsulfoxid, bei Temperaturen zwischen 20 und 80°C umsetzt (vergleiche hierzu auch die Angaben in J. Am. Chem. Soc. *87*, 1363-1364 (1965), oder

β) mit Trimethylsulfonium-methylsulfat der Formel:

$$\overset{(+)}{[(CH_3)_3S]} \quad \overset{(-)}{CH_3SO_4} \qquad (XI)$$

in an sich bekannter Weise in Gegenwart eines inerten organischen Lösungsmittels, wie z. B. Acetonitril, und in Gegenwart einer Base, wie z. B. Natriummethylat, bei Temperaturen zwischen 0 bis 60°C, vorzugsweise bei Raumtemperatur, umsetzt (vergleiche auch die Angaben in Heterocycles *8*, 397, 1977).

Die so erhaltenen Oxirane der Formel (V) können gegebenenfalls ohne Isolierung direkt weiter umgesetzt werden.

Die bei der Herstellung der Oxirane der Formel (V) als Ausgangsstoffe benötigten Ketone der Formel (IX) sind bekannt (vergleiche z. B. DE-PS Nr. 2201063, DE-OS Nr. 2632603, DE-OS Nr. 2632602, DE-OS Nr. 2635664, DE-OS Nr. 2635666, DE-OS Nr. 2705678, DE-OS Nr. 2918894, DE-OS Nr. 2918893, DE-PS Nr. 2705678, DE-OS Nr. 2737489), bzw. sind sie Gegenstand eigener älterer Patentanmeldungen, die noch nicht veröffentlicht sind (vergleiche die Europäischen Patentansmeldungen EP-A Nr. 42980 vom 1.6.1981, EP-A Nr. 65203 vom 5.5.82, EP-A Nr. 56933 vom 7.1.1982), bzw. lassen sie sich nach dem im Prinzip bekannten Verfahren herstellen.

Das bei der Verfahrensvariante (α) benötigte Dimethyloxosulfoniummethylid der Formel (X) ist ebenfalls bekannt (vergleiche J. Am. Chem. Soc. *87*, 1363-1364, 1965). Es wird bei der obigen Umsetzung in frisch zubereitetem Zustand verarbeitet,indem man es *in situ* durch Umsetzung von Trimethylsulfoxosulfoniumiodid mit Natriumhydrid bzw. Natriumamid in Gegenwart eines Verdünnungsmittels erzeugt.

Das bei der Verfahrensvariante (β) benötigte Trimethylsulfonium-methylsulfat der Formel (XI) ist ebenfalls bekannt (vgl. Heterocycles *8*, 397, 1977). Es wird bei der obigen Umsetzung ebenfalls in frisch hergestelltem Zustand eingesetzt,indem man es durch Reaktion von Dimethylsulfid mit Dimethylsulfat *in situ* erzeugt.

Die auch als Ausgangsstoffe zur Herstellung der 1-Hydroxyalkyl-azole der Formel (IV) zu verwendenden Azolylmethyloxirane der Formel (VII) sind noch nicht bekannt. Sie sind jedoch teilweise Gegenstand einer eigenen älteren Anmeldung, die noch nicht veröffentlicht ist (vergleiche die Patentanmeldung EP-A Nr. 61051 vom 8.3.1981), teilweise sind sie Gegenstand einer parallelen Patentanmeldung, bzw. können sie in allgemein bekannte Art und Weise erhalten werden, indem man Azolo-ketone der Formel:

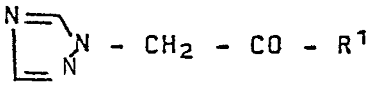 (XII)

in welcher:

$R^1$ die oben angegebene Bedeutung hat, entsprechend den oben angegebenen Verfahrensvarianten α und β epoxidiert.

Die Azolo-ketone der Formel (XII) sind bekannt (vergleiche DE-OS Nr. 2431407, DE-OS Nr. 2638470, DE-OS Nr. 2820361), bzw. sind sie Gegenstand einer eigenen älteren Patentanmeldung, die noch nicht veröffentlicht ist (vergleiche die Patentanmeldung EP-A Nr. 54865 vom 11.12.1980), bzw. lassen sie sich nach im Prinzip bekannten Verfahren herstellen.

Die ausserdem als Ausgangsstoffe zur Herstellung der 1-Hydroxyalkyl-azole der Formel (IV) zu verwendenden Azole der Formel (VI) bzw. (Thio)-Phenole der Formel (VIII) sind allgemein bekannte Verbindungen der organischen Chemie. Die weiterhin für das erfindungsgemässe Verfahren als Ausgangsstoffe zu verwendenden Halogenide sind durch die Formel (III) allgemein definiert. In dieser Formel steht $R^2$ vorzugsweise für die Bedeutungen, die bereits im Zusammenhang mit der Beschreibung der erfindungsgemässen Stoffe der Formel (I) vorzugsweise für diesen Substituenten genannt wurden. Hal steht vorzugsweise für Chlor, Brom oder Iod.

Die Halogenide der Formel (III) sind allgemein bekannte Verbindungen der organischen Chemie.

Als Beispiele für die substituierten 1-Hydroxyalkyl-azole der Formel (IV), die den erfindungsgemäss als Ausgangsstoffe zu verwendenden Alkoholaten der Formel (II) zugrunde liegen, seien genannt:

*Tabelle I*

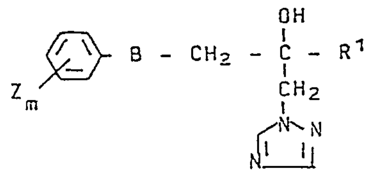 (IV)

| $Z_m$ | B | $R^1$ |
|---|---|---|
| 4-Cl | O | $C(CH_3)_3$ |
| 4-Cl | $CH_2$ | $C(CH_3)_3$ |
| 4-Cl,2-$CH_3$ | O | $C(CH_3)_3$ |
| 2,4-$Cl_2$ | O | $C(CH_3)_3$ |
| 4-$CH_3$ | O | $C(CH_3)_3$ |
| 2-$CH_3$ | O | $C(CH_3)_3$ |
| 4-F | $CH_2$ | $C(CH_3)_3$ |
| 4-Cl | O | 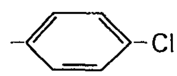 |
| 4-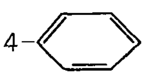 | O | $C(CH_3)_3$ |
| 2-Cl | O | $C(CH_3)_3$ |
| 2,4-$Cl_2$ | $CH_2$ | $C(CH_3)_3$ |
| 2-$CH_3$ | $CH_2$ | $C(CH_3)_3$ |
| 4-Cl | O | 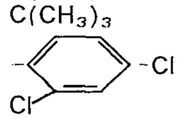 |
| 4-F | O | $C(CH_3)_3$ |
| 3-Cl | O | $C(CH_3)_3$ |
| 2-Cl,4-F | O | $C(CH_3)_3$ |
| 3,4-$Cl_2$ | O | $C(CH_3)_3$ |
| 4-$CH_3$ | $CH_2$ | $C(CH_3)_3$ |
| 4-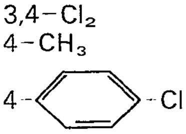 | O | $C(CH_3)_3$ |
| 4-$OCH_3$ | O | $C(CH_3)_3$ |
| 4-$C(CH_3)_3$ | O | $C(CH_3)_3$ |
| 4-$OCF_3$ | O | $C(CH_3)_3$ |
| 4-F | O | $-C(CH_3)_2CH_2F$ |
| 4-Cl | O | $-C(CH_3)_2CH_2OCH_3$ |
| 4-Cl | S | $C(CH_3)_3$ |

*Tabelle I* (Fortzetzung)

| $Z_m$ | B | $R^1$ |
|---|---|---|
| 4- 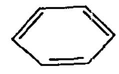 | O | $-C(CH_3)_2CH=CH_2$ |
| 4-$CH_3$ | O | $-C(CH_3)_3CH_2F$ |
| 4-Cl | O | $-C(CH_3)_2CH_2F$ |
| 4-Cl,2-$CH_3$ | O | $-C(CH_3)_2CH_2F$ |
| 2,4-$Cl_2$ | O | $-C(CH_3)_2CH_2F$ |
| 4-Cl | S | $-C(CH_3)_2CH_2F$ |
| 2-Cl | S | $-C(CH_3)_2CH_2F$ |
| 3,4-$Cl_2$ | S | $-C(CH_3)_2CH_2F$ |
| 4-Cl | O | $-C(CH_2F)_2CH_3$ |
| 2,4-$Cl_2$ | O | $-C(CH_2F)_2CH_3$ |
| 4-Cl,2-$CH_3$ | O | $-C(CH_2F)_2CH_3$ |
| 2,4-$Cl_2$ | O | $-C(CH_3)_2CH_2OCH_3$ |
| 4-Cl,2-$CH_3$ | O | $-C(CH_3)_2CH_2OCH_3$ |
| 4-Cl | O | $-C(CH_3)_2CH_2OC_2H_5$ |
| 4-Cl | O | $-C(CH_3)_2CH_2O-$ 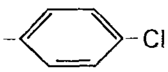 |
| 4-F | O | $-C(CH_3)_2CH_2OCH_3$ |
| 2,4-$Cl_2$ | O | $-C(CH_3)_2$ 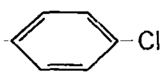 |
| 4-Cl | O | $-C(CH_3)_2CH=CH_2$ |
| 2,4-$Cl_2$ | O | $-C(CH_3)_2CH=CH_2$ |
| 4-F | O | $-C(CH_3)_2CH=CH_2$ |
| 4-Cl | $CH_2$ | $-C(CH_3)_2CH_2F$ |
| 2,4-$Cl_2$ | $CH_2$ | $-C(CH_3)_2CH_2F$ |
| 2,4-$Cl_2$ | $CH_2$ | $-C(CH_2F)_2CH_3$ |
| 4-Cl | $CH_2$ | $-C(CH_2F)_2CH_3$ |
| 4-Cl | $CH_2$ | $-C(CH_3)_2CH_2O-$ 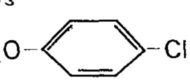 |
| 4-Cl | $CH_2$ | $-C(CH_3)_2O-$ 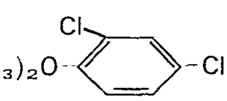 |
| 3,4-$Cl_2$ | S | $-C(CH_3)_3$ |

Zur vorliegenden Erfindung gehören pharmazeutische Zubereitungen, die neben nicht toxischen, inerten pharmazeutisch geeigneten Trägerstoffen einen oder mehrere erfindungsgemässe Wirkstoffe enthalten oder die aus einem oder mehreren erfindungsgemässen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, dass die Zubereitungen in Form einzelner Teile, z. B. Tabletten, Dragées, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder ½, ⅓ oder ¼ einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragées, Kapseln, Pillen, Granulate, Suppositorien, Lösungen, Suspensionen und Emulsionen, Pasten, Salben, Gele, Cremes, Lotions, Puder und Sprays genannt.

Tabletten, Dragées, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quaternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragées, Kapseln, Pillen und Granulaten können mit den üblichen gegebenenfalls Opakisierungsmittel enthaltenden Überzügen und Hüllen versehen sein und auch so zusammengesetzt sein, dass sie den oder die Wirkstoffe nur oder bevorzugt in einem bestimmten Teil des Intestinaltraktes, gegebenenfalls verzögert, abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnussöl, Meiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruch- und geschmackverbessernde Zusätze, z. B. Pfefferminzöl und Eukalyptusöl und Süssmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können ausser den erfindungsgemässen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemässen Wirkstoffe sowie von pharmazeutischen Zubereitungen, die einen oder mehrere erfindungsgemässe Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemässen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muss. Die Festlegung der jeweils erforderlichen optimalen Dosierung und Applikationsart der Wirkstoffe kann durch jeden Fachmann aufgrund seines Fachwissens leicht erfolgen.

*Herstellungsbeispiele*

*Beispiel 1:*

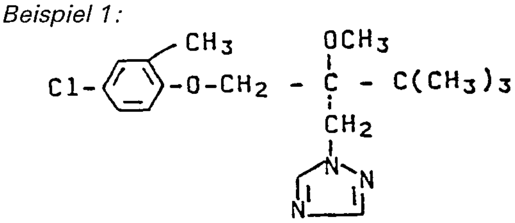

27 g (0,9 mol) 80%-iges Natriumhydrid werden bei Raumtemperatur portionsweise in eine Lösung von 290 g (0,86 mol) 2-(4-Chlor-2-methyl-phenoxy-methyl)-3,3-dimethyl-1(1,2,4-triazol-1-yl)-2-butanol in 1600 ml absolutem Dioxan eingetragen. Man lässt das Gemisch 4 Stunden bei Raumtemperatur nachrühren und tropft anschliessend 141,9 g (1 mol) Iodmethan zu. Das Reaktionsgemisch wird 12 Stunden bei 40°C gerührt und danach erneut potionsweise mit 10 g (0,33 mol) 80%-igem Natriumhydrid versetzt. Nach dreistündigem Rühren bei Raumtemperatur werden 57 g (0,4 mol) Iodmethan zugegeben und das Reaktionsgemisch 72 Stunden bei Raumtemperatur nachgerührt. Die Suspension wird filtriert, das Filtrat eingeengt, der ölige Rückstand in Dichlormethan aufgenommen, zweimal mit Wasser gewaschen, über Natriumsulfat getrocknet und eingeengt. Der Rückstand wird in Aceton mit 1,5-Naphthalindisulfonsäure versetzt, das ausfallende Salz wird abgesaugt, mit Aceton gewaschen, in Dichlormethan suspendiert und mit gesättigter Natriumhydrogencarbonatlösung versetzt. Die organische Phase wird abgetrennt, gewaschen und eingeengt. Man erhält 155 g (54% der Theorie) 2-(4-Chlor-2-methyl-phenoxymethyl)-3,3-dimethyl-2-methoxy-1-(1,2,4-triazol-1-yl)-butan als hellgelbes Öl vom Brechungsindex $n_D^{20}$ = 1,5390.

In analoger Weise und entsprechend dem erfindungsgemässen Verfahren werden die nachfolgenden Verbindungen der allgemeinen Formel:

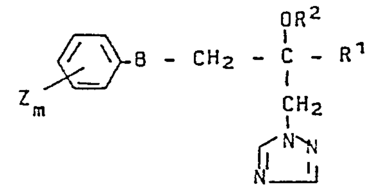 (I)

erhalten:

*Tabelle II*

| Bsp. Nr: | $Z_m$ | B | $R^1$ | $R^2$ | Fp (°C) bzw. $n_D^{20}$ |
|---|---|---|---|---|---|
| 2 | 4-Cl | O | $C(CH_3)_3$ | $CH_3$ | 198-203 (×HCl) |
| 3 | 2,4-$Cl_2$ | O | $C(CH_3)_3$ | $CH_3$ | 1,5382 |
| 4 | 4-Cl | $CH_2$ | $C(CH_3)_3$ | $CH_3$ | 1,5354 |
| 5 | 4-F | $CH_2$ | $C(CH_3)_3$ | $CH_3$ | 1,5212 |

*Verwendungsbeispiele*

In den nachfolgenden Verwendungsbeispielen werden die nachstehend angegebenen Substanzen als Vergleichsverbindungen eingesetzt:

(A) 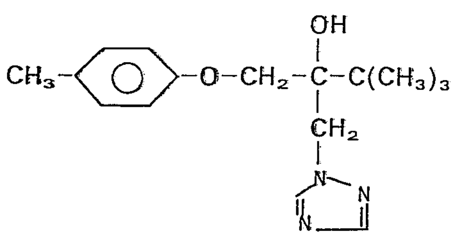

(B) 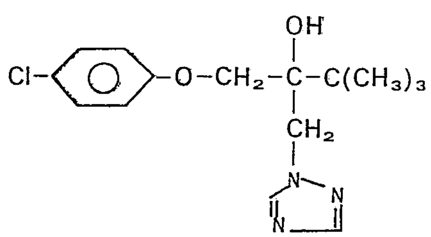

(C) 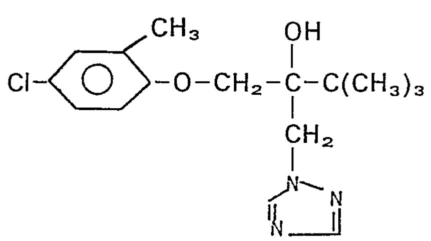

(D) 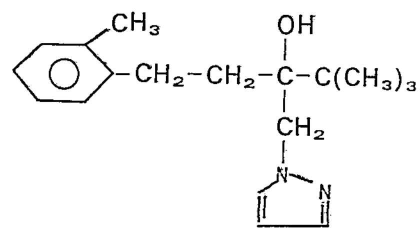

*Beispiel A:*

*Antimykotische* in-vitro- *Wirksamkeit*

Versuchsbeschreibung:

Die *in-vitro*-Prüfungen wurden im Reihenverdünnungstest mit Keiminokula von durchschnittlich 5 × $10^4$ Keimen/ml Substrat durchgeführt. Als Nährmedium dienten:

a) für Dermatophyten und Schimmelpilze: Sabouraud's milieu d'épreuve

b) für Hefen: Fleischextrakt-Traubenzucker-Bouillon.

Die Bebrütungstemperatur betrug 20°C, die Bebrütungsdauer lag bei 24 bis 96 Stunden bei Hefen und 96 Stunden bei Dermatophyten und Schimmelpilzen.

In diesem Test zeigen beispielsweise die Verbindungen 1, 3, 4 und 5 eine bessere antimykotische Wirkung als die aus dem Stand der Technik bekannten Verbindungen (A), (B), (C) und (D).

*Tabelle A*

*Antimykotische* in-vitro- *Wirksamkeit*

| Wirkstoff | MHK-Werte Trichophyton mentagr. | Nährmedium (ml) Candida albicans | Aspergillus fumig. | Microsporum canis | Troulopsis glabrata |
|---|---|---|---|---|---|
| (A) (bekannt) | <1 | 4 | 8 | 4 | 64 |
| (B) (bekannt) | 8 | <1 | 64 | 16 | 2 |
| (C) (bekannt) | <1 | 8 | 2 | 4 | 16 |
| (D) (bekannt) | 8 | 2 | >64 | 16 | 16 |
| *Verbindungen gemäss Herstellungsbeispielen* | | | | | |
| 1 | <1 | <1 | 2 | < 1 | < 1 |
| 3 | <1 | 2 | 4 | < 1 | 2 |
| 4 | <1 | <1 | < 1 | < 1 | 4 |
| 5 | <1 | <1 | 2 | < 1 | 2 |

*Beispiel B:*

*Antimykotische* in-vivo- *Wirksamkeit (oral) bei Mäusecandidose*

*Versuchsbeschreibung:*

Mäuse vom Typ SPF-$CF_1$ wurden intravenös mit 1 - $2 \times 10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50-100 mg/kg Körpergewicht der Präparate oral behandelt.

*Ergebnis:*

Unbehandelte Tiere starben 3 bis 6 Tage post infektionem. Die Überlebensrate am 6. Tag post infektionem betrug bei unbehandelten Kontrolltieren etwa 5%.

In diesem Test zeigten z. B. die erfindungsgemässen Verbindungen 1, 2, 3, 4 und 5 eine bessere Wirkung als die aus dem Stand der Technik bekannten Verbindungen (A), (B), (C) und (D).

*Tabelle B*

*Antimykotische* in-vivo- *Wirksamkeit (oral) bei Mäusecandidose*

| Wirkstoff | Wirkung |
|---|---|
| (A) (bekannt) | k.W. |
| (B) (bekannt) | k.W. |
| (C) (bekannt) | k.W. |
| (D) (bekannt) | k.W. |
| *Verbindungen gemäss Herstellungsbeispielen* | |
| 1 | +++ |
| 2 | ++++ |
| 3 | +++ |
| 4 | +++++ |
| 5 | ++++ |

+++++ = sehr gute Wirkung = 90% Überlebende am 6. Tag p.i.
++++ = gute Wirkung = 80% Überlebende am 6. Tag p.i.
+++ = Wirkung = 60% Überlebende am 6. Tag p.i.
++ = schwache Wirkung = 40% Überlebende am 6. Tag p.i.
+ = Spur Wirkung = unter 40% Überlebende am 6. Tag p.i.
k.W. = keine Wirkung

*Beispiel C:*

*Antimikrobielle* in-vivo- *Wirksamkeit (lokal) am Modell der experimentellen Meerschweinchen-Trichophytie*

*Versuchsbeschreibung:*

Weisse Mäuse der Rasse Pirbright-white wurden auf dem geschorenen, nicht skarifizierten Rücken mit einer Mikro- und Makrokonidien-Suspension von Trichophyton mentagrophytes infiziert.

Die infizierten Tiere wurden, beginnend mit dem 3. Tag p.i., 1 × täglich mit einer 15%-igen Lösung der erfindungsgemässen Präparate (in Dimethylsulfoxid: Glycerin = 1:4) lokal behandelt.

*Ergebnis:*

Bei unbehandelten Tieren entwickelte sich innerhalb 12 Tagen p.i. das typische Bild einer Dermatophytose mit Rötung, Schuppung und Haarausfall bis zum totalen Integument-Defekt an der Infektionsstelle.

In diesem Test zeigen z. B. die erfindungsgemässen Verbindungen 1, 2, 3, 4 und 5 gute Wirkung.

*Tabelle C*

*Antimykotische* in-vivo- *Wirksamkeit (lokal) am Modell der experimentellen Meerschweinchen-Trichophytie*

| Wirkstoff | Wirkung |
|---|---|
| *Verbindungen gemäss Herstellungsbeispielen* | |
| 1 | ++++ |
| 2 | ++++ |
| 3 | ++++ |
| 4 | ++++ |
| 5 | ++++ |

+++++ = sehr gute Wirkung = keine Infektionszeichen am 12.-15. Tag p.i.
++++ = gute Wirkung = geringe Rötung, vereinzelt Schuppen
+++ = Wirkung = Rötung, Schuppung, ohne Haarausfall
++ = schwache Wirkung = Rötung, Schuppung, Haarausfall
+ = Spur Wirkung = flächiger Haarausfall, entzündliche Hautreaktion

*Beispiel D/Formulierungen:*

*1. Lösung:*

| | |
|---|---|
| Wirkstoff gemäss Formel (I) | 10 g |
| Alkohol, rein (96%-ig) | 300 g |
| Isopropylmyristat | 526 g |
| | 836 g |

*2. Creme:*

| | |
|---|---|
| Wirkstoff gemäss Formel (I) | 10 g |
| Arlacel 60 | 20 g |
| (Sorbitan-monostearat) Tween 60 | 15 g |
| (Polyoxyethylen(20)-sorbitan-monostearat) Walrat, künstlich | 30 g |
| (Mischung von Estern von gesättigten Fettsäuren $C_{14}$-$C_{18}$ und Fettalkoholen $C_{14}$-$C_{18}$) Lanette O | 100 g |
| (Gemisch aus Cetyl-Alkohol und Stearyl-Alkohol) Entanol G | 135 g |
| (2-Octyl-dodecanol) Benzylalkohol | 10 g |
| Wasser, entmineralisiert | 680 g |
| | 1000 g |

**Patentansprüche**

1. Antimykotisches Mittel, gekennzeichnet durch einen Gehalt an mindestens einem Etherderivat eines substituierten 1-Hydroxyalkyl-azolyl-Derivates der allgemeinen Formel:

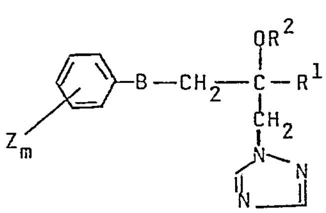

(I)

in welcher:

B für Sauerstoff, Schwefel oder die $CH_2$-Gruppe steht;

$R^1$ für die Gruppierungen

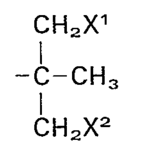

und

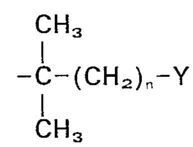

steht, oder für gegebenenfalls durch Alkyl mit 1 bis 2 Kohlenstoffatomen substituiertes Cycloalkyl mit 3 bis 7 Kohlenstoffatomen und für gegebenenfalls substituiertes Phenyl steht, wobei als Substituenten genannt seien:

Halogen, Alkyl mit 1 bis 4 Kohlenstoffatomen sowie Halogenalkyl mit 1 bis 2 Kohlenstoff- und 1 bis 5 gleichen oder verschiedenen Halogenatomen;

$X^1$ für Wasserstoff oder Halogen steht;,

$X^2$ für Halogen steht;

Y für Alkyl, Alkoxy, Alkylthio, Halogenalkoxy, Halogenalkylthio, Alkenyl mit bis zu 6 Kohlenstoffatomen, Alkoxycarbonyl mit 1 bis 4 Kohlenstoffatomen im Alkylteil, Cyano sowie für gegebenenfalls substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten genannt seien:

Halogen, Alkyl mit 1 bis 4 Kohlenstoffatomen; Alkoxy und Alkylthio mit jeweils 1 bis 2 Kohlenstoffatomen; Halogenalkyl, Halogenalkoxy und Halogenalkylthio mit jeweils 1 bis 2 Kohlenstoffatomen und 1 bis 5 gleichen oder verschiedenen Halogenatomen, Cyclohexyl, Dialkylamino mit 1 bis 4 Kohlenstoffatomen in jedem Alkylteil, Nitro, Cyano sowie Alkoxycarbonyl mit 1 bis 4 Kohlenstoffatomen im Alkylteil;

n für die Zahlen 0, 1 oder 2 steht;

Z für Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoffatomen und 1 bis 5 gleichen oder verschiedenen Halogenatomen sowie für jeweils gegebenenfalls durch Halogen und Alkyl mit 1 bis 2 Kohlenstoffatomen substituiertes Phenyl, Phenoxy und Phenylalkyl sowie Phenylalkoxy mit 1 bis 2 Kohlenstoffatomen im Alkylteil bzw. im Alkoxyteil steht;

$R^2$ für geradkettiges oder verzweigtes Alkyl mit 1 bis 4 Kohlenstoffatomen, für geradkettiges oder verzweigtes Alkenyl und Alkinyl mit jeweils 2 bis 4 Kohlenstoffatomen sowie für gegebenenfalls substituiertes Phenyl oder Benzyl, wobei als Phenylsubstituenten die bei Y bereits genannten in Frage kommen, und

m für die Zahlen 0, 1, 2 oder 3 steht;

sowie deren Säureadditionssalze.

2. Antimykotisches Mittel nach Anspruch 1, gekennzeichnet durch einen Gehalt an mindestens einer Verbindung der Formel (I) im Anspruch 1, in der

$R^1$ für geradkettiges oder verzweigtes Alkyl mit 1 bis 4 Kohlenstoffatomen steht, und

B, $R^2$, Z und m für die in Anspruch 1 genannten Bedeutungen stehen.

3. Antimykotisches Mittel nach Anspruch 1, gekennzeichnet durch einen Gehalt an mindestens einer Verbindung der Formel (I) in Anspruch 1, in der

$R^1$ für die Gruppierungen:

$$-\overset{\displaystyle CH_2X^1}{\underset{\displaystyle CH_2X^2}{C}}-CH_3 \quad \text{und} \quad -\overset{\displaystyle CH_3}{\underset{\displaystyle CH_3}{C}}-(CH_2)_n-Y$$

Benzyl oder Benzyloxy steht;

$R^2$ für Methyl, Ethyl, n-Propyl, Isopropyl, n-Butyl, Isobutyl, Allyl, Propargyl sowie für gegebenenfalls substituiertes Phenyl oder Benzyl steht, wobei als Phenylsubstituenten genannt seien: Fluor, Chlor, Methyl, Ethyl, Methoxy, Methylthio, Trifluormethyl, Trifluormethoxy und Trifluormethylthio, und

B und m für die in Anspruch 1 angegebene Bedeutung stehen.

4. Antimykotisches Mittel nach Anspruch 1, gekennzeichnet durch einen Gehalt an mindestens einer Verbindung der Formel (I) in Anspruch 1, in der

$R^1$ für tert.-Butyl, Isopropyl oder Methyl steht; und

B, $R^2$, Z und m für die in Anspruch 3 genannten Bedeutungen stehen.

5. Antimykotisches Mittel nach Anspruch 1, gekennzeichnet durch einen Gehalt an 2-(4-Chlor-2-méthyl-phenoxymethyl)-3,3-dimethyl-2-methoxy-1-(1,2,4-triazol-1-yl)-Butan.

6. Verfahren zur Herstellung eines antimykotischen Mittels, dadurch gekennzeichnet, dass man Etherderivate von substituierten 1-Hydroxyalkyl-azolyl-Derivaten gemäss Formel (I) in Anspruch 1 mit inerten, nichttoxischen, pharmazeutisch geeigneten Trägerstoffen vermischt.

## Claims

1. Antimycotic agent, characterised in that it contains at least one ether derivative of a substituted 1-hydroxyalkyl-azolyl derivative of the general formula:

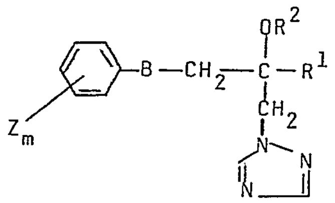

(I)

in which

B represents oxygen, sulphur or the $CH_2$ group;

$R^1$ represents the groupings:

$$-\overset{\displaystyle CH_2X^1}{\underset{\displaystyle CH_2X^2}{C}}-CH_3 \quad \text{and} \quad -\overset{\displaystyle CH_3}{\underset{\displaystyle CH_3}{C}}-(CH_2)_n-Y$$

or represents cycloalkyl which has 3 to 7 carbon atoms and is optionally substituted by alkyl with 1 to 2 carbon atoms, and optionally substituted phenyl, the following being mentioned as substituents: halogen, alkyl with 1 to 4 carbon atoms and halogenoalkyl with 1 to 2 carbon atoms and 1 to 5 identical or different halogen atoms;

$X^1$ represents hydrogen or halogen,

$X^2$ represents halogen;

Y represents alkyl, alkoxy, alkylthio, halogenoalkoxy, halogenoalkylthio, alkenyl with up to 6 carbon atoms, alkoxycarbonyl with 1 to 4 carbon atoms in the alkyl part, cyano and optionally substituted phenyl, phenoxy, phenylthio, phenylalkoxy with 1 to 4 carbon atoms in the alkyl part and phenylalkylthio with 1 to 4 carbon atoms in the alkyl part, the following being mentioned as phenyl substituents in each case: halogen, alkyl with 1 to 4 carbon atoms; alkoxy and alkylthio with in each case 1 to 2 carbon atoms; halogenoalkyl, halogenoalkoxy and halogenoalkylthio with in each case 1 to 2 carbon atoms and 1 to 5 identical or different halogen atoms, cyclohexyl, dialkylamino with 1 to 4 carbon atoms in each alkyl part, nitro, cyano and alkoxycarbonyl with 1 to 4 carbon atoms in the alkyl part;

n represents the numbers 0, 1 or 2;

Z represents halogen, alkyl with 1 to 4 carbon atoms, cycloalkyl with 5 to 7 carbon atoms, alkoxy and alkylthio with in each case 1 to 4 carbon atoms, halogenoalkyl and halogenoalkoxy and halogenoalkylthio with in each case 1 to 2 carbon atoms and 1 to 5 identical or different halogen atoms, and phenyl, phenoxy and phenylalkyl and phenylalkoxy with 1 to 2 carbon atoms in the alkyl part or in the alkoxy part, each of which is optionally substituted by halogen and alkyl with 1 to 2 carbon atoms;

$R^2$ represents straight-chain or branched alkyl with 1 to 4 carbon atoms, straight-chain or branched alkenyl and alkinyl with in each case 2 to 4 carbon atoms and optionally substituted phenyl or benzyl, possible phenyl substituents being those already mentioned under Y, and

m represents the numbers 0, 1, 2 or 3;

and acid addition salts thereof.

2. Antimycotic agent according to Claim 1, characterised in that it contains at least one compound of the formula (I) in Claim 1, in which

$R^1$ represents staight-chain or branched alkyl with 1 to 4 carbon atoms, and

B, $R^2$, Z and m represent the meanings mentioned in Claim 1.

3. Antimycotic agent according to Claim 1, characterised in that it contains at least one compound of the formula (I) in Claim 1, in which

$R^1$ represents the groupings:

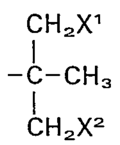 and 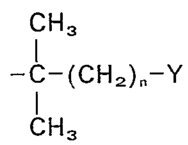

benzyl or benzyloxy;

$R^2$ represents methyl, ethyl, n-propyl, isopropyl, n-butyl, isobutyl, allyl, propargyl, and optionally substituted phenyl or benzyl, the following being mentioned as phenyl substituents: fluorine, chlorine, methyl, ethyl, methoxy, methylthio, trifluoromethyl, trifluoromethoxy and trifluoromethylthio, and

B and m represent the meaning given in Claim 1.

4. Antimycotic agent according to Claim 1, characterised in that it contains at least one compound of the formula (I) in Claim 1, in which

$R^1$ represents tert.-butyl, isopropyl or methyl, and

B, $R^2$, Z and m represent the meanings mentioned in Claim 3.

5. Antimycotic agent according to Claim 1, characterised in that it contains 2-(4-chloro-2-methyl-phenoxymethyl)-3,3-dimethyl-2-methoxy-1-(1,2,4-triazol-1-yl)-butane.

6. Process for the preparation of an antimycotic agent, characterised in that ether derivatives of substituted 1-hydroxyalkyl-azolyl derivatives according to formula (I) in Claim 1 are mixed with inert, non-toxic, pharmaceutically suitable excipients.

## Revendications

1. Composition antimycosique, caractérisée par une teneur en au moins un éther d'un dérivé de 1-hydroxyalkylazolyle substitué de formule générale:

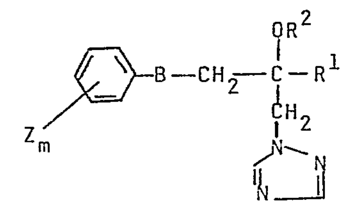

(I)

dans laquelle:

B désigne l'oxygène, le soufre ou le groupe $CH_2$;

$R^1$ représente les groupements:

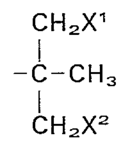 et 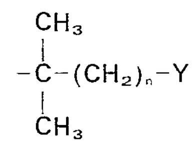

ou bien un groupe cycloalkyle de 3 à 7 atomes de carbone éventuellement substitué par un radical alkyle ayant 1 ou 2 atomes de carbone, et un groupe phényle éventuellement substitué, et on mentionne alors comme substituants: un halogène, un groupe alkyle ayant 1 à 4 atomes de carbone ainsi qu'un groupe halogénalkyle ayant 1 ou 2 atomes de carbone et 1 à 5 atomes d'halogènes égaux ou différents;

$X^1$ est l'hydrogène ou un halogène,

$X^2$ est un halogène;

Y est un groupe alkyl, alcoxy, alkylthio, halogénalcoxy, halogénalkylthio, alcényle ayant jusqu'à 6 atomes de carbone, alcoxycarbonyle ayant 1 à 4 atomes de carbone dans la partie alkyle, cyano ainsi qu'un groupe phényle éventuellement substitué, phénoxy, phénylthio, phénylalcoxy ayant 1 à 4 atomes de carbone dans la partie alkyle et phénylalkylthio ayant 1 à 4 atomes de carbone dans la partie alkyle, et on mentionne alors comme substituants du groupe phényle dans chaque cas: un halogène, un substituant alkyle ayant 1 à 4 atomes de carbone; des substituants alcoxy et alkylthio ayant chacun 1 ou 2 atomes de carbone, des substituants halogénalkyle, halogénalcoxy et halogénalkylthio ayant chacun 1 ou 2 atomes de carbone et 1 à 5 atomes d'halogènes égaux ou différents, un substituant cyclohexyle, dialkylamino ayant 1 à 4 atomes de carbone dans chaque partie alkyle, nitro, cyano, ainsi qu'un substituant alcoxycarbonyle ayant 1 à 4 atomes de carbone dans la partie alkyle;

n représente les nombres 0, 1 ou 2;

Z représente un halogène, un groupe alkyle ayant 1 à 4 atomes de carbone, cycloalkyle ayant 5 à 7 atomes de carbone, alcoxy et alkylthio ayant chacun 1 à 4 atomes de carbone, halogénalkyle et halogénalcoxy ainsi qu'halogénalkylthio ayant dans chaque cas 1 ou 2 atomes de carbone et 1 à 5 atomes d'halogènes égaux ou différents, ainsi que des groupes phényle, phénoxy et phénylalkyle de même que phénylalcoxy avec 1 ou 2 atomes de carbone dans la partie alkyle et respectivement dans la partie alcoxy, éventuellement substitués chacun par un halogène et un substituant alkyle ayant 1 ou 2 atomes de carbone;

$R^2$ est un groupe alkyle à chaîne droite ou ramifié ayant 1 à 4 atomes de carbone, un groupe alcényle et un groupe alcynyle à chaîne droite ou ramifiés ayant chacun 2 à 4 atomes de carbone ainsi qu'un groupe phényle ou benzyle éventuellement substitué, et on considère alors comme substituants du noyau phényle ceux qui ont déjà été mentionnés dans le cas de Y, et

m représente les nombres 0, 1, 2 ou 3;

ainsi que leurs sels d'addition d'acides.

2. Composition antimycosique suivant la revendication 1, caractérisée par une teneur en au moins un composé de formule (I) suivant la revendication 1, dans laquelle:

$R^1$ est un groupe alkyle à chaîne droite ou ramifié ayant 1 à 4 atomes de carbone, et

B, $R^2$, Z et m ont les définitions mentionnées dans la revendication 1.

3. Composition antimycosique suivant la revendication 1, caractérisée par une teneur en au moins un composé de formule (I) suivant la revendication 1, dans laquelle:

$R^1$ représente les groupements:

$$\begin{array}{c} CH_2X^1 \\ | \\ -C-CH_3 \\ | \\ CH_2X^2 \end{array} \quad \text{et} \quad \begin{array}{c} CH_3 \\ | \\ -C-(CH_2)_n-Y \\ | \\ CH_3 \end{array}$$

benzyle ou benzyloxy;

$R^2$ est un groupe méthyle, éthyle, n-propyle, isopropyle, n-butyle, isobutyle, allyle, propargyle ainsi qu'un groupe phényle ou benzyle éventuellement substitué, et on mentionne alors comme substituants du noyau phényle: le fluor, le chlore, les groupes méthyle, éthyle, méthoxy, méthylthio, trifluorométhyle, trifluorométhoxy et trifluorométhylthio, et

B et m ont la définition indiquée dans la revendication 1.

4. Composition antimycosique suivant la revendication 1, caractérisée par une teneur en au moins un composé de formule (I) suivant la revendication 1, dans laquelle:

$R^1$ est un groupe tertiobutyle, isopropyle ou méthyle, et

B, $R^2$, Z et m ont la définition mentionnée dans la revendication 3.

5. Composition antimycosique suivant la revendication 1, caractérisée par une teneur en 2-(4-chloro-2-méthylphénoxyméthyl)-3,3-diméthyl-2-méthoxy-1-(1,2,4-triazole-1-yl)-butane.

6. Procédé de préparation d'une composition antimycosique, caractérisé en ce qu'on mélange des éthers de dérivés de 1-hydroxyalkylazolyle substitués de formule (I) suivant la revendication 1 avec des supports inertes non toxiques acceptables du point de vue pharmaceutique.